# EUROPEAN PATENT SPECIFICATION

(11) **EP 2 164 964 B1**
(45) Date of publication and mention of the grant of the patent: **07.09.2011**
(21) Application number: 08758308.4
(22) Date of filing: 04.07.2008
(51) Int. Cl.: C12N 15/11, A61K 48/00

(54) **DEHYDRATED CHITOSAN NANOPARTICLES**
DEHYDRATISIERTE CHITOSAN-NANOPARTIKEL
NANOPARTICULES DESHYDRATEES DE CHITOSANE

(30) Priority: 06.07.2007 DK 200701004
(43) Date of publication of application: 24.03.2010
(73) Proprietor: Aarhus Universitet, 8000 Aarhus C (DK)
(72) Inventor: KJEMS, Jørgen, DK-8240 Risskov (DK); HOWARD, Kenneth Alan, DK-8200 Aarhus N (DK); BESENBACHER, Flemming, DK-8210 Aarhus V (DK); ANDERSEN, Morten Østergaard, DK-8200 Aarhus N (DK)
(74) Representative: Plougmann & Vingtoft A/S
(86) International application number: PCT/DK2008/050171
(87) International publication number: WO 2009/006905

(56) References cited:
- WO-A-98/01160
- DATABASE WPI Week 200380 Thomson Scientific, London, GB; AN 2003-857506 XP002498522 & JP 2003 265171 A (KOIKE KAGAKU KK) 24 September 2003 (2003-09-24)
- MAO HAI-QUAN ET AL: "Chitosan-DNA nanoparticles as gene carriers: Synthesis, characterization and transfection efficiency" JOURNAL OF CONTROLLED RELEASE, vol. 70, no. 3, 23 February 2001 (2001-02-23), pages 399-421, XP002498519 ISSN: 0168-3659
- DATABASE MEDLINE [Online] US NATIONAL LIBRARY OF MEDICINE (NLM), BETHESDA, MD, US; June 2004 (2004-06), BOZKIR ASUMAN ET AL: "Chitosan-DNA nanoparticles: effect on DNA integrity, bacterial transformation and transfection efficiency." XP002498520 Database accession no. NLM15512779 & JOURNAL OF DRUG TARGETING JUN 2004, vol. 12, no. 5, June 2004 (2004-06), pages 281-288, ISSN: 1061-186X
- LIU ET AL: "The influence of polymeric properties on chitosan/siRNA nanoparticle formulation and gene silencing" BIOMATERIALS, ELSEVIER SCIENCE PUBLISHERS BV., BARKING, GB, vol. 28, no. 6, 28 November 2006 (2006-11-28), pages 1280-1288, XP005782068 ISSN: 0142-9612
- DATABASE MEDLINE [Online] US NATIONAL LIBRARY OF MEDICINE (NLM), BETHESDA, MD, US; February 2008 (2008-02), ANDERSEN MORTEN ET AL: "Delivery of siRNA from lyophilized polymeric surfaces." XP002498521 Database accession no. NLM17950838 & BIOMATERIALS FEB 2008, vol. 29, no. 4, February 2008 (2008-02), pages 506-512, ISSN: 0142-9612

### SEQUENCE LISTING

<110> Arhus Universitet
   Kjems, Jorgen
   Besenbacher, Flemming
   Oestergaard Andersen, Morten
   Howard, Kenneth Alan
<120> Dehydrated chitosan nanoparticles
<130> 42397PC01
<160> 8
<170> PatentIn version 3.5
<210> 1
   <211> 21
   <212> RNA
   <213> Homo Sapiens
<400> 1
   gacguaaacg gccacaaguu c 21
<210> 2
   <211> 20
   <212> RNA
   <213> Homo Sapiens
<400> 2
   acuuguggcc guuuacgucg 20
<210> 3
   <211> 20
   <212> RNA
   <213> Homo Sapiens
<400> 3
   gacguuagac ugacaaguuc 20
<210> 4
   <211> 23
   <212> RNA
   <213> Homo Sapiens
<400> 4
   acuugguguc cagucuaagu cgc 23
<210> 5
   <211> 25
   <212> RNA
   <213> Homo Sapiens
<400> 5
   gucucagccu cuucucauuc cugcu 25
<210> 6
   <211> 26
   <212> RNA
   <213> Homo Sapiens
<400> 6
   uacagagucg ggaagaguaa ggacga 26
<210> 7
   <211> 25
   <212> RNA
   <213> Homo Sapiens
<400> 7
   cuuccucucu uucucucccu uguga 25
<210> 8
   <211> 27
   <212> RNA
   <213> Homo Sapiens
<400> 8
   aggaaggaga gaaagagagg gaacacu 27

## Description

### Background

Since the discovery of the RNA interference (RNAi) mechanism small interfering RNAs (siRNAs) have proved valuable tools for assaying gene function and potential therapeutics. RNAi occurs when cytoplasmic double stranded (ds) RNA guides a RNA nuclease silencing complex (RISC) to cleave homologous sequences in a messenger RNA molecule resulting in gene silencing. Carrier systems are a requirement for efficient delivery of siRNA by overcoming extracellular and intracellular barriers. Lipid (lipoplexes) and polycationic polymer-based (polyplexes) non-viral systems are attractive candidates due to the immunogenicity and safety issues associated with viral delivery.

In genomics and drug target validation the trend leans towards high throughput loss of function screening utilizing RNAi. Different approaches utilizing libraries of sequences delivered as chemically synthesised siRNAs or short-hairpin siRNA expression plasmids by viral vectors have been developed. Non-viral delivery of siRNAs offers greater control of siRNA concentration and predictable toxicity patterns. In the high throughput screening systems using siRNA the common strategy is to mix siRNA with a cationic lipid transfection agent to form lipoplexes, these can then be added together with gelatine in order to facilitate spotting of the reagent onto slides for analysis.

Unfortunately, the non-viral transfection agent itself often alter gene expression, therefore, any comprehensive screening method should use several delivery vehicles to reduce this effect on the obtained results. A combination of current and new systems of spottable and storable siRNA formulations would, therefore, offer improved certainty of the results obtained in high throughput siRNA screenings.

siRNA is also being developed as a therapeutic drug to silence genes implicated in diseases. In order for nucleotide-based drugs to attain widespread clinical use there is a need for formulations with controlled dosage, activity and side effects. Aqueous formulations of non-viral complexes have a problem fulfilling these requirements since they rapidly deteriorate by aggregation if stored at 25 °C, 4 °C or -20 °C.

Since both siRNA screenings and siRNA therapeutics could benefit from storable, locally active siRNA formulations there is a need for development of such systems. Chitosan nanoparticles comprising SiRNA are known from Biomaterials, 28, Feb 2007, 1280-8.

### Summary of the invention

The present invention provides a dehydrated nanoparticle comprising a SiRNA and chitosan. The siRNA is capable of modulating the expression of a target gene. Such nanoparticles are useful in genomics and drug target validation. Moreover, they are useful as medicaments.

### Brief description of the drawings

Figure 1. Effect of sucrose on chitosan/siRNA knockdown of eGFP in H1299 cells. Chitosan/siRNA samples were added in the presence of variable sucrose concentrations into the wells of 24-well plates at the indicated concentrations of siRNA. The plates were then freeze dried, seeded with H1299 cell line and transfected for 48 hours. Fluorescence was measured by flowcytometry and normalized to an untransfected control. All samples were run in triplicates.
Figure 2. Knockdown of eGFP in H1299 cells with of Trans-IT TKO/siRNA and chitosan/siRNA.
   Chitosan/siRNA samples containing 10% sucrose and Trans-IT TKO/Mirus/siRNA were added at the indicated concentrations into the wells of a 24-well plates. The plates were then freeze dried, seeded with H1299 cells and transfected for 48 hours. Fluorescence was measured by flowcytometry and normalized to an untransfected control. All samples were run in triplicates.

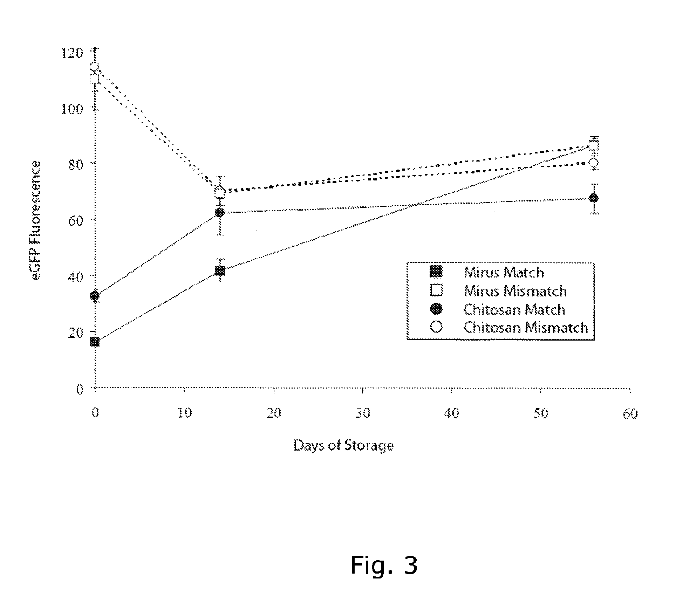

Figure 3. Effect of storage on activity of Chitosan/siRNA and Mirus/siRNA. Plated freeze dried chitosan/siRNA (10% sucrose) and Trans-IT TKO/siRNA formulations were stored at 25°C in moderate sunlight. At different time points H1299 cells were plated and knock down of eGFP measured. The fluorescence levels were normalized to an untransfected control.

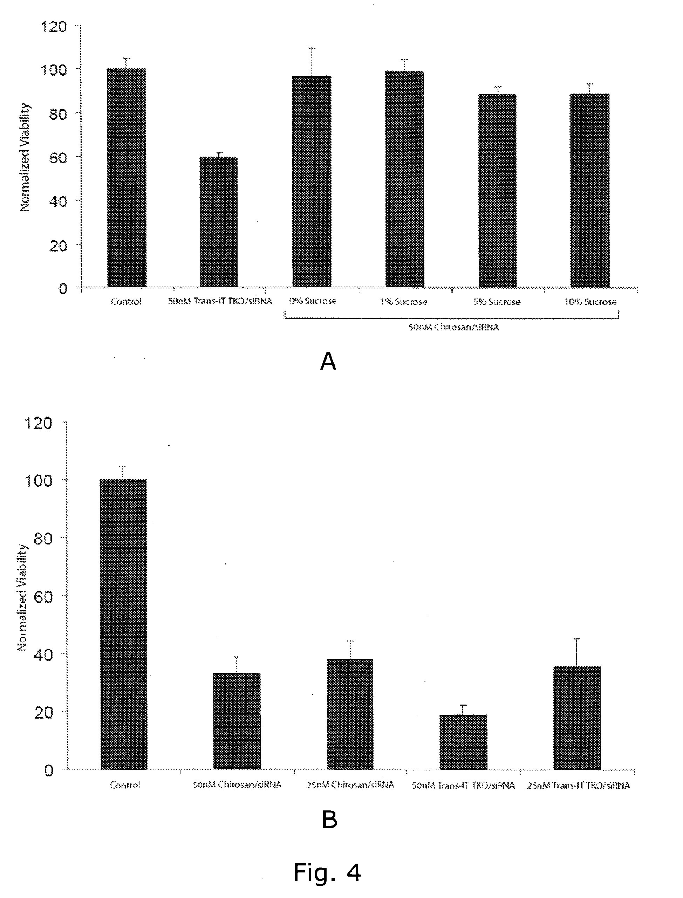

Figure 4. Viability of H1299 and Raw cells subjected to chitosan/siRNA and Trans-IT TKO/siRNA. Chitosan/siRNA samples with or without sucrose and Trans-IT TKO/siRNA were freeze dried into wells on 24 well plates. The plates were seeded with H1299 cells (Figure 4A) or Raw cells (Figure 4B) and transfected for 48 hours. 20ul aqueous cytotoxicity solution was then added to the wells and after 90 minutes absorbance was read. Viability values were normalized to the untransfected control. All samples in triplicates.

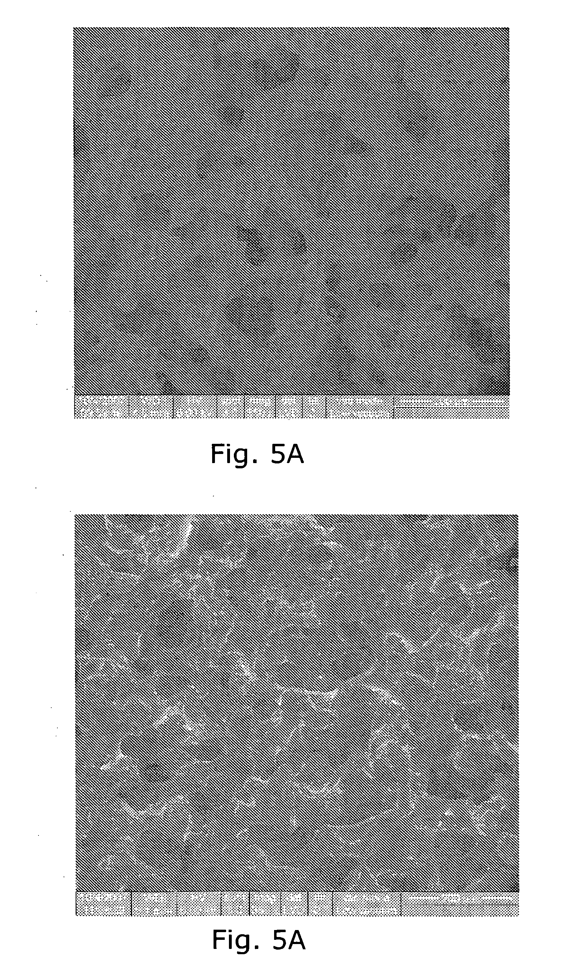

Figure 5. Coating of tissue engineering scaffolds with chitosan/siRNA containing 10% sucrose. Poly-ε-caprolactone scaffolds were coated with chitosan/siRNA particles. Scaffolds without (Figure 5A) and with (Figure 5B) chitosan/siRNA particle coating were visualized using scanning electron microscopy. See example 2 for details.

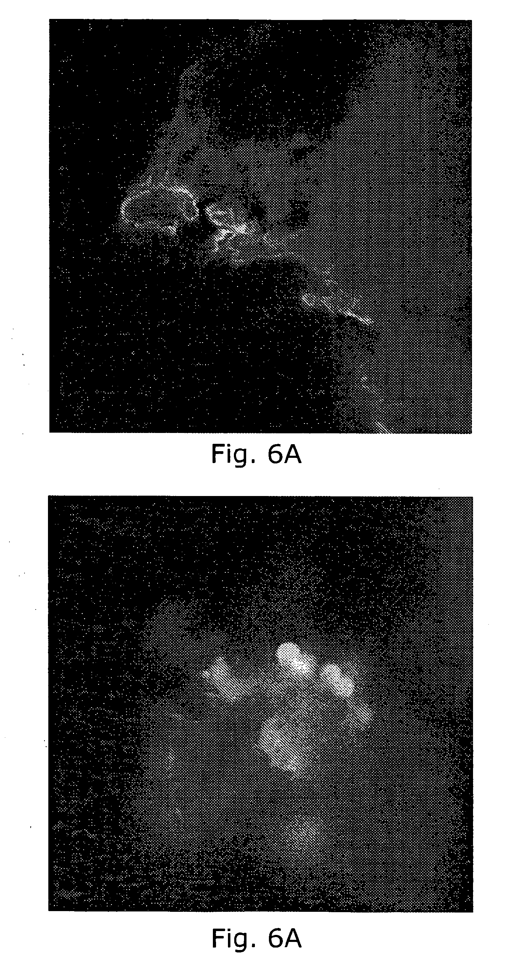

Figure 6. Coated scaffolds were also added water and the presence of chitosan particles made with fluorescent siRNA was investigated using confocal fluorescence microscopy (Figure 6A). Finally rat mesenchymal stem cells were seeded on the scaffolds and their uptake of siRNA was evaluated after 24 hours using confocal fluorescence microscopy (Figure 6B). Representative pictures presented. See example 2 for details.

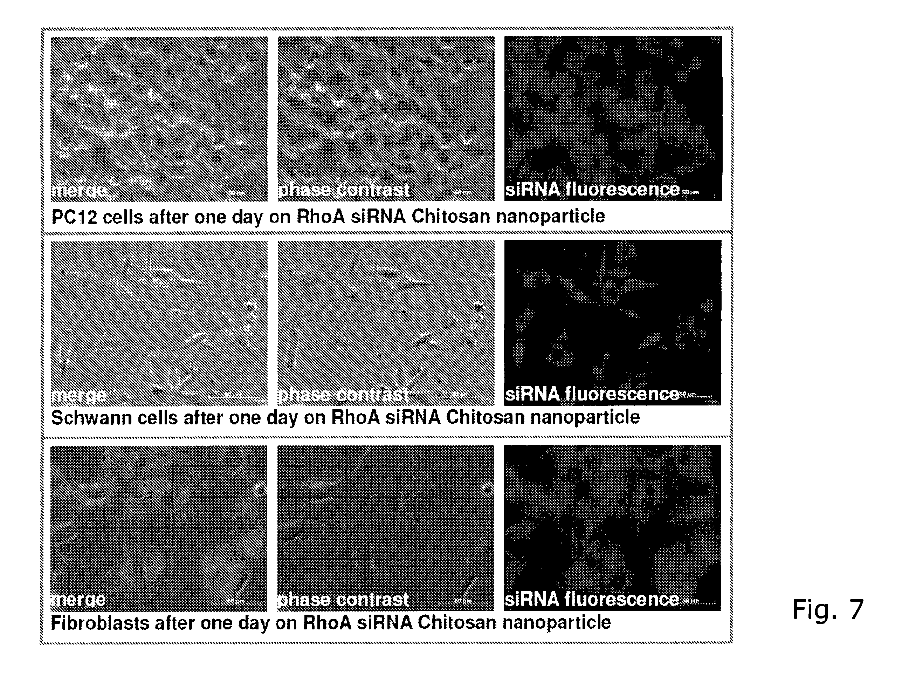

Figure 7. Uptake of chitosan/siRNA in various neural cell lines. Chitosan/siRNA samples containing 10% sucrose were added into the wells of a 24-well plates. The plates were then freeze dried, seeded with cells and transfected for 24 hours. The uptake of fluorescent siRNA was investigated using fluorescent microscopy. All samples were run in triplicates, representative pictures presented.

### Disclosure of the invention

In a first aspect, the invention provides a dehydrated nanoparticle comprising a siRNA and chitosan. The dehydrated nanoparticle is advantageous e.g. because it prolongs period of time in which the SiRNA nanoparticle retains its activity. Moreover, in some embodiments it allows storage at room temperature. As will be clear from the embodiments described below, the nanoparticles are also favourable because they can be immobilized on solid supports, which enable new applications of the nanoparticles.

The term chitosan as used herein has the same meaning as generally in the art. I.e. chitosan refers to a linear polysaccharide composed of randomly distributed β-(1-4)-linked D-glucosamine (deacetylated unit) and N-acetyl-D-glucosamine (acetylated unit). Chitosan is typically produced by deacetylation of chitin, which is the structural element in the exoskeleton of crustaceans (crabs, shrimp, etc.). The degree of deacetylation in commercial chitosans is in the range 60-100 %. In a preferred embodiment, also derivatives of chitosan may be used, e.g. provided by chemical modification of chitosan.

Preferably, the water content of the dehydrated nanoparticle is less than 5% (w/w of water/nanoparticle). In other embodiments, the water content is less than 4%, 3%, 2% and 1%, respectively. Most preferred is a water content of less than 0,1 %.

The nucleic acid of the nanoparticle is a RNA molecule capable of mediating RNA interference to silence a target gene. The RNA is a small interfering RNA (siRNA).

siRNAs are typically double stranded RNA complexes comprising a passenger strand and a guide strand, the guide strand directing RNA interference to a target gene. Various embodiments (blunt ended, 3'-overhang, etc.) of siRNAs are known to the skilled man.

The dehydrated nanoparticle of the invention may be prepared by any drying activity, which preserves the activity of the nucleic acid. Obvious parameters that may be adjusted are pressure and temperature and a preferred drying method is lyophilisation. Thus, preferably drying is done at a temperature and pressure, where the water of the nanoparticle is solid.

In another preferred embodiment, the nanoparticle is prepared by spray drying.

The nanoparticle of the invention may further comprise a lyoprotectant. Preferably, the lyoprotectant is selected from the group consisting of sucrose, glucose, inulin and trehalose. The amount of lyoprotectant is preferably less 20 % and even more preferred less than 10% (w/w). In yet another embodiment, it is preferred that the amount of lyoprotectant is between 5 % and 10%.

The nanoparticle of the invention may also further comprise a spotting agent, such as gelatine or collagen or any other extracellular matrix component or derivative thereof as well as agarose or any other carbohydrate. This is favourable e.g. where the nanoparticle is to be dried onto a surface.

The nanoparticle may also comprise a cell adhesion substance selected from the group consisting of fibronectin, RGD peptides, antibodies, aptamers, lipids, poly-L-Lysine, carbohydrates and extracellular matrix components or derivatives thereof.

Also a targeting moiety such as transferrin, an antibody or and aptamer may be comprised in the nanoparticle.

The nanoparticle of the invention may also be included in a material that can control siRNA release such as a larger controlled release particle, an implantable drug depot or a biodegradable scaffold for tissue engineering. In such embodiment, the particles are embedded within a degrading matrix and released as the matrix degrades. Preferably the matrix is a biodegradable polymer such as poly-ε-caprolactone, polylacticacid, polyglycolicacid or poly(lactic-co-glycolic)acid. The nanoparticle of the invention may be dried onto a surface. Preferably, the surface is part of a culture well, vial, an implant for tissue engineering or a prosthetic implant.

Thus, it can be e.g. lyophilized directly into cell culture dishes or onto surfaces and used later in one step by simply adding cell suspension directly.

In one embodiment where the nanoparticle has been dried onto an implant, the siRNA targets a cytokine, preferably a pro-inflammatory cytokine and most preferably cytokine TNF-alfa, to reduce the immune response towards the implant.

In another embodiment where the nanoparticle has been dried onto an implant, the siRNA targets a mRNA that encodes a protein that represses or enhances the differentiation of stem cells, to either enhance or repress one or more specific differentiation pathways respectively.

Method of forming the nanoparticle to be dehydrated.

Preferably, the nanoparticle of the invention is prepared by a method comprising
a. Providing a chitosan solution
b. Providing an siRNA solution
c. Mixing the solution of step a with the solution of step b
d. Incubating the solution of step c under conditions of complex formation such that chitosan/siRNA nanoparticles form.

In one embodiment, the chitosan/siRNA nanoparticle comprises an initial crosslinker, such as polyphosphate.

In another preferred embodiment of the method of preparing a chitosan siRNA nanoparticle, the chitosan does not comprise an initial crosslinker.

The term initial cross linker is used to for a crosslinker that is added to chitosan to form a particle, before the siRNA molecule is added. Typically in the art, e.g. when chitosan/plasmid nanoparticles are formed, the particles are preformed using an initial crosslinker such as polyphosphate. Thus, it is believed that the structure and activity of a particle formed without an initial crosslinker differ from that of a particle formed with an initial crosslinker. In particular, the use of an initial crosslinker seems to imply that the siRNA will be distributed at the surface of the preformed particle, whereas when using the siRNA as crosslinker, the siRNA will be distributed evenly through the particle. An even distribution is expected to have a positive effect on the biostability of the siRNA molecules of the nanoparticle, as they will be less accessible to RNases.

Moreover, the omission of the initial crosslinker provides a more facile method of preparation. Instead of a two-step method, where the particles are formed first and then the siRNA is added, a one-step method is provided in which the siRNA and chitosan is mixed to form nanoparticles directly.

Thus, in a preferred embodiment, the siRNA functions as a crosslinker in the formation of a nanoparticle. In other words, the siRNA the formactive component.

### Concentrations

### siRNA concentration

In one of embodiment of the method of preparing a chitosan/siRNA nanoparticle, the siRNA solution comprises siRNA at a concentration selected from the group consisting of at least 5 µM, at least 10 µM, at least 20 µM, at least 30 µM, at least 40 µM, at least 50 µM, at least 60 µM, at least 70 µM, at least 80 µM, at least 90 µM and at least 100 µM.

### Chitosan concentration

In another embodiment, the chitosan solution comprises chitosan at a concentration from the group consisting of at least 50 µg/ml, at least 60 µg/ml, at least 70 µg/ml, at least 80 µg/ml, at least 90 µg/ml, at least 100 µg/ml, at least 110 µg/ml, at least 120 µg/ml, at least 130 µg/ml, at least 140 µg/ml, at least 150 µg/ml, at least 160 µg/ml, at least 170 µg/ml, at least 180 µg/ml, at least 190 µg/ml, at least 200 µg/ml, at least 250 µg/ml, at least 500 µg/ml, at least 750 µg/ml and at least 1000 µg/ml.

### Degree of deacetylation

Preferably, the chitosan has a relatively high degree of deacetylation. Thus, in one embodiment, the chitosan has a degree of deacetylation of selected from the group consisting of at least 60%, least 65%, least 70%, least 75%, least 80%, least 85% and at least 95%.

### Molecular weight of chitosan

The molecular weight of the chitosan is preferably more than 10 kDa. In another embodiment, the molecular weight is more than 50 kDa and even more preferred is a molecular weight of more than 100 kDa.

Chitosan samples with a molecular weight in the range of 100-170 kDa and a high (greater than 70 %) deacetylation degree are particular favourable.

### N:P ratio

The above mentioned parameters can all be used to control the characteristics of the formed nanoparticle. Another important parameter is the so-called N:P ratio, defined herein as the ratio of chitosan amino groups (N) to RNA phosphate groups (P).

In a preferred embodiment, the nanoparticle is formed at a N:P ratio larger than 50. Experiments document that increasing the N:P ratio, leads to larger particles. In other embodiments, the N:P ratio is selected from the group consisting of a N:P ratio larger than 60, larger than 70, larger than 80, larger than 90, larger than 100 and larger than 150.

In this preferred embodiment, wherein the N:P ratio is larger than 70, the siRNA solution comprises siRNA at a concentration lower than 100 µM, such as lower than 90 µM, lower than 80 µM, lower than 70 µM, lower than 60 µM, lower than 50 µM, lower than 40 µM, lower than 30 µM, lower than 20 µM, lower than 10 µM, lower than 5 µM or lower than 1 µM.

When employing a high N:P ratio and a low RNA concentration, nanoparticles can be formed that comprises loosely bound chitosan.

As the degree of loosely bound chitosan is dependent on both the concentration of siRNA in the siRNA solution and on the N: P ratio, the skilled worker will appreciate how to manipulate these parameters to create nanoparticles with loosely bound chitosan. E.g. a high siRNA concentration of the siRNA solution may be used, if also the N:P ratio is kept high, i.e. a high concentration of chitosan is used.

In one embodiment, the nanoparticle comprising loosely bound chitosan has a high N:P ratio.

A nanoparticle with loosely bound chitosan is of interest e.g. to improve mucosal delivery. Therefore, in one embodiment, the nanoparticle with loosely bound chitosan is for mucosal delivery, in particular pulmonary delivery.

A nanoparticle particle with a discrete character is of interest e.g. for systemic delivery. Such a particle can also be formed by controlling various parameters involved in the method of forming the nanoparticle. Particularly, a low N:P ratio favours the formation of a discrete nanoparticle. As mentioned above, the concentration of siRNA and chitosan can be varied while maintaining a reasonably constant N:P ratio.

### Concentrated Method vs. N:P ratio

In this embodiment, the N:P ratio is lower than 70 such as but not limited to a N:P ratio lower than 60, lower than 50, lower than 40, lower than 30, lower than 20 or lower than 10, respectively.

In one embodiment, the nanoparticle of discrete character has a low N:P ratio. In another embodiment, the concentration of the siRNA solution is at least 100 µM, such as but no limited to at least 250 µM, at least 200 µM, at least 150 µM, at least 90 µM, at least 80 µM, at least 70 µM, at least 60 µM or at least 50 µM, respectively.

Using a high concentration of siRNA in the siRNA solution turns out to have several advantages. As outlined in the examples section, when the particles are formed using a siRNA solution with a concentration of 250 µM, the particles are more discrete and monodispersed, as compared to particles formed using a pre-diluted siRNA solution of 20 µM (as can be seen from the PDI measurements in table 3). Moreover, it surprisingly turns out that the nanoparticles formed using the concentrated siRNA solution have a more specific effect, i.e. they do not give rise to any non-specific knockdown, which may be the case for particles formed using a siRNA solution with a lower concentration of siRNA.

Furthermore, using a high concentration of siRNA in the siRNA solution allows particle formation at a low pH such as ph 4.5, which in turn makes the particles more stable. Using a slightly higher pH of 5.5 is also possible. A pH of 5.5 may decrease detrimental effects of acetate buffer on cell viability.

Additionally, using a high concentration of siRNA in the siRNA solution means that the amount of siRNA in particles increase, which decreases the amount of particle solution that has to be administered to a cell or organism.

In another embodiment, the chitosan concentration is less than 250 µg/ml.

In a particular preferred embodiment, the chitosan concentration is less than 250 µg/ml, while the siRNA concentration is higher than 100 µM.

By controlling the concentrations of siRNA and chitosan, and thereby the N: P ratio, also the size of the particles can be controlled, (as documented in the examples section. Thus, in one embodiment, the size of the particle is between 10 and 200 nM.

In another embodiment, the formed particles are discrete in form and have a polydispersity index lower than 0,4.

Another aspect of the invention is a surface comprising a nanoparticle of the invention, wherein the nanoparticle has been dried onto the surface.

The surface may be part of a culture well, vial, an implant for tissue engineering or a prosthetic implant

Still another aspect of the invention is a powder of dehydrated nanoparticles of the invention and a further aspect is a pharmaceutical composition comprising the dehydrated nanoparticle of the invention. In one embodiment this powder resuspends completely when added water or buffer or any other liquid and gives particles of the same composition, charge, size, transfection efficiency and toxicity as before they were dried. Preferably this liquid can then be used for therapeutical purposes.

Since the RNA part of the nanoparticle may be used to modulate the activity of a particular target, a nanoparticle comprising the siRNA can be used for treatment. The sequence of the siRNA can be designed such as to sequence specifically target an mRNA. Thus, if it is known that a particular protein is causing disease or unwanted condition, the expression of the protein may be downregulated by using a siRNA that target the mRNA encoding the protein. Thereby, the disease or condition may be alleviated. This is very well known in the field of small interfering RNA (and microRNAs). In general, antisense RNA can be used to specifically target an mRNA. Aptamers will typically have a protein as target, which in general terms also make them suited for therapeutics.

Nanoparticles of the invention may be useful in mucosal delivery in carrying out a treatment.

Mucosal delivery may be selected from the group of delivery to the respiratory tract (upper and/or lower respiratory tract) oral delivery (oesophagus, stomach, small and large intestine) or genitourinary tract (e.g. anus, vagina) delivery.

Another aspect of the invention is a dehydrated nanoparticle of the invention for use a medicament.

And still another aspect of the invention is the use of the dehydrated nanoparticle of the invention for the preparation of a medicament for treatment selected from the group of treatment of cancer, treatment of viral infections such as influenza, respiratory synthetic virus and bacterial infections e.g. tuberculosis and treatments of inflammatory conditions such as arthritis, Crohn's and hay fever.

### Examples

### Example 1

### Introduction

In this study, we describe a method for producing gene silencing-active lyophilised cationic polymer (chitosan) siRNA formulations. We demonstrate specific and efficient knockdown of Enhanced Green Fluorescent Protein (EGFP) in H1299 human lung carcinoma cells transfected in plates pre-coated with a chitosan/siRNA formulation containing sucrose as lyoprotectant (~ 70 %). This method removes the necessity for both siRNA reconstitution immediately prior to use and addition onto cells. Furthermore, silencing activity of the plate was maintained in the period studied (~ 2 Months) when stored at room temperature.

Higher cell viability was observed using the chitosan system compared to the lipid formulation. Silencing of the proinflammatory cytokine Tumour Necrosis Factor (TNF-α) was also demonstrated in the RAW macrophage cell line using the lyophilised polymer/siRNA system suggesting that the coating can improve the biocompatibility of medical implants.

This work describes an efficient gene silencing methodology using freeze-dried formulations with potential applications as a high throughput screening tool for gene function, biocompatible medical implant components and longer shelf-life therapeutics.

Lyophilized material, however, can be stored at room temperature without losing activity for extended periods and reconstituted in a reproducible manner immediately prior to use.

An alternative application is the incorporation of lyophilized siRNA complexes into tissue engineering scaffolds such as has been done with lyophilized DNA/polyethylenimine (PEI) complexes.

Unfortunately most scaffolds induce host inflammatory responses after implantation. Lyophilized siRNA formulations directed against proinflammatory cytokines such as Tumour Necrosis Factor (TNF-α) could be used to coat implants as a method to reduce inflammatory responses.

### Materials & Methods

### siRNA sequences

eGFP targeted siRNA (sense strand: 5'-GACGUAAACGGCCACAAGUUC-3', antisense strand: 3'-GCUGCAUUUGCCGGUGUUCA-5') and eGFP mismatch siRNA (sense strand: 5'-GACGUUAGACUGACAAGUUC-3', antisense strand: 3'-CGCUGAAUCUGACCUGUGGUUCA-5'), the TNF-α Dicer substrate (DsiRNAs) 27mer targeted siRNA (sense strand: 5'-GUCUCAGCCUCUUCUCAUUCCUGCT-3', antisense 3'-AGCAGGAAUGAGAAGGGCUGAGACAU-5',) and mismatch (sense strand: 5' CUUCCUCUCUUUCUCUCCCUUGUGA-3', antisense strand: 3'-UCACAAGGGAGAGAAAGAGAGGAAGGA-5'.

### Chitosan/siRNA nanoparticles and TransIT-TKO Formulation

Chitosan/siRNA particles were prepared as described previously, briefly chitosan is dissolved over night at pH 4.3 in acetate buffer which is adjusted to pH 5.5 with 1M NaOH. 1 ml of 0.2 mg/ml chitosan is then added 20 µl 20 µM siRNA (N:P ratio 50) while stirring and after 1 hour the mixture is added water or sucrose at varying concentrations and plated. The hydrodynamic radiuses of the nanoparticles were measured using Photon correlation spectroscopy (PCS). Trans-IT TKO/siRNA particles were made according to the manufacturer's protocol. Briefly 3 µl Trans-IT TKO reagent and 3 µl 5 µM siRNA were mixed separately with 25 µl RPMI media and then added together. After 20 minutes the mixture is plated.

When referring to the amount of plated siRNA complex we used the concentration (in nMs) of the siRNA when the sample is added in 250 µl Water, a 50 nM plating refers to a pre-lyophilization volume of 38,4 µl of Chitosan/siRNA particles or 50 µl Trans-IT TKO/siRNA particles. All plated formulations were frozen to -20 °C before 48 hour lyophilization at 80 mT and -20 °C. After lyophilization the plated formulations were stored at 25 °C on the bench.

### Cell culture and transfections

We used a H1299 human lung carcinoma cell line stably expressing enhanced green fluorescent protein and a TNF-α expressing RAW murine macrophage cell line. Both cell lines were maintained at 37 °C at 5 % CO₂ and 100 % humidity. Cells were seeded at densities of 4 x 10⁵ cells/ml in fresh RPMI media containing 10 % foetal bovine serum, 1 % Penicillin & streptomycin and 0.5 % G418 into wells coated with lyophilized transfection reagent. After 24 hours the media was replaced with fresh media either 500 µl media (24 well plates) or 100 µl media (96 well plates). After 24 additional hours the transfection was completed. All transfection samples were done in triplicates.

### Cellular cytotoxicity of lyophilised formulations

Transfection samples were evaluated for cellular cytotoxicity using a tetrazolium-based viability assay. The wells were added 20 µl aqueous cytotoxicity solution and after 1.5 hours the absorbance was read at 562 nm. The absorbance of the blank wells was subtracted from the absorbance of the sample wells. All cytotoxicity samples were done in triplicates.

### Flow cytometric determination of cellular fluorescence

Transfected H1299 cells were harvested by a standard trypsination protocol. The harvested cells were washed with PBS and fixed in PBS containing 1 % paraformaldehyde. The EGFP cell fluorescence was measured using a flow cytometer. A histogram plot with log green fluorescence intensity on the x axis and cell number on the y axis was used to define median fluorescence intensity of the main cell population defined by scatter properties (forward and side scatter, not shown).

### TNF- α ELISA

10 µl 1 µg/ml lipopolysaccharide(LPS) was added to the wells containing RAW cells and stored for 5 hours. The supernatants were assayed for TNF-α using an enzyme-linked immunosorbent assay (ELISA). Maxisorp plates were coated overnight at room temperature with 100 µl of goat anti-mouse TNF-α capture antibody in a concentration of 2 µg/ml in coating buffer (15 mM Na₂CO₃, 35 mM NaHCO₃, 0.02 % NaN₃ [pH 9.6]). After blocking for at least 1 h at room temperature with 300 µl of 1 % bovine serum albumin (BSA) in blocking buffer (phosphate-buffered saline with 5 % sucrose and 0.05 % NaN3 [pH 7.4]), successive culture supernatants or recombinant murine TNF-α was added to the wells (100 µl each) and incubated overnight at 4 °C. Subsequently, wells were incubated at room temperature for 2 h with 100 µl biotinylated, goat anti-mouse TNF-α detection antibody (150 ng/ml). Streptavidin-horseradish peroxidase diluted at 1:200 in TBS with 0.1 % BSA was added, and the mixture was incubated for 20 min. For color development, we added H₂O₂ and tetramethylbenzidine and plates were incubated in the dark for an appropriate amount of time. The color reaction was stopped with 50 µl 5 % H₂SO₄, and the absorbance was measured at 450 nm with 570 nm as a reference. Between each step, the plates were washed three times with phosphate-buffered saline-0.05 % Tween 20, pH 7.4. Finally concentrations of TNF-alpha were normalized to the measured viability of each well.

### Results

### Hydrodynamic radius determination of chitosan/siRNA nanoparticles

In order to study the effect of lyophilization on the morphology of the chitosan particles we studied the changes in the hydrodynamic size in the presence of 0 or 10 % sucrose using PCS (Table 1).

**Table 1: Hydrodynamic diameter of particles before and after lyophilization. 500ul Chitosan/siRNA particles were added either 100ul 10% aqueous sucrose or 100ul water. Hydrodynamic diameters were measured with PCS before and after freeze drying (samples were reconstituted in 600ul water by pipetting up and down). N/A refers to the particle size being above the detection limit of 6000nm. Measurements were done three times at 25°C. Standard deviation is listed in brackets.**

| Chitosan/eGFP siRNA Particles | Before Lyophilization | After Lyophilization |
|---|---|---|
| 0% Sucrose | 126,0 (26,1) | N/A |
| 10% Sucrose | 168,8 (13,7) | 142,4 (25,4) |

For each sample particle sizes were measured three times at 25 °C. Listed are the average peak size and standard deviations of these three measurements. Both in the presence and absence of sucrose samples show a hydrodynamic size of ~150 nm before lyophilization. After lyophilization the sample containing 10 % sucrose yielded particles with approximately the same size (~150 nm) while the sample without sucrose dissolved to give visible large aggregates that could not be measure by PCS.

### Knockdown of eGFP in H1299 Cells

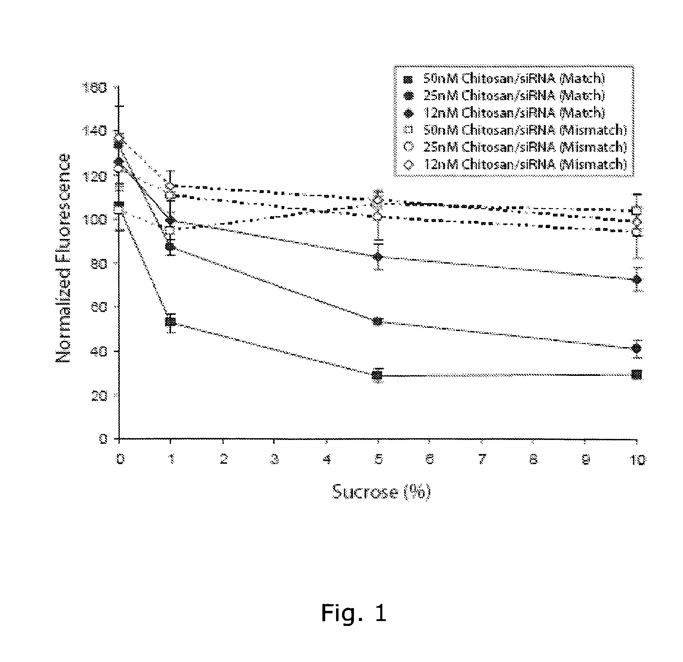

We analyzed the ability of the freeze-dried chitosan/siRNA nanoparticle formulation to knockdown stably expressed eGFP in the H1299 cell line (Fig. 1). The knockdown efficiency increased with the siRNA concentration and dependent on the presence of sucrose as lyoprotectant. At lower siRNA concentration (<=25 nM) the highest knock down was obtained at relatively high sucrose concentrations (60 % knock down at 10 % sucrose) whereas, at high siRNA concentration (50 nM) 5 % sucrose was sufficient to reach maximum knock down efficiency (70 %).

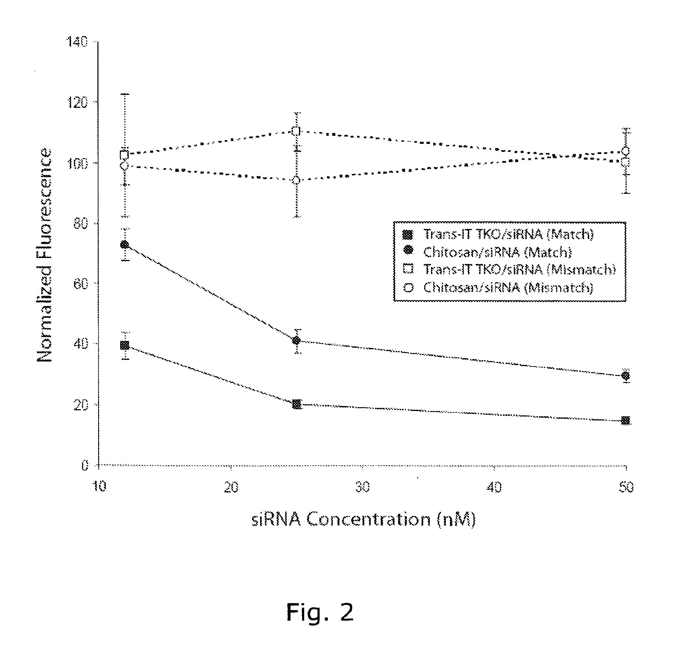

We then compared the freeze dried chitosan system to the freeze dried Trans-IT TKO system (Fig. 2). Freeze dried Mirus/siRNA and chitosan/siRNA both silenced eGFP at all concentrations tested, the silencing efficiency diminished with concentration. The unspecific knockdown, measured using the mismatched control, was low in both systems at all siRNA concentrations tested (<10 %; Fig. 2).

### Stability of freeze-dried chitosan/siRNA nanoparticles on storage

The effect of storage on the transfection efficiency of plated formulations was investigated over a 2 month period. At different time points H1299 cells were plated and knockdown efficiency determined (Fig. 3). Specific silencing was observed during entire period studied with chitosan/siRNA.

### Knockdown of TNF-a in RA W cells

To evaluate the ability of the freeze-dried formulations to silence the expression of inflammatory genes TNF-α knockdown in macrophages was investigated (Table 2). After LPS stimulation, non-transfected macrophages produced large amounts of TNF-α (stimulated 11-fold: 1100 pg/ml compared to 96 pg/ml in the control). The induction was reduced to 7 and 8.5-fold in the presence of 50 and 25 nM chitosan/siRNA, respectively. The TNF-α production was almost completely abolished using Trans-IT TKO at both siRNA concentrations. Interestingly, there was a large difference in the unspecific knockdown of TNF-α expression between the systems. Whereas chitosan weakly induced additional TNF-α production (10-20 %) Trans-IT TKO reduced it to nearly around the detection limit of the assay at 50 nM and to 50 % at 25 nM siRNA. Hence, chitosan particles appear to yield a more specific TNF-α knock down than particles formed with Trans-IT TKO.

**Table 2: Knockdown of TNF-α in Macrophages with Trans-IT TKO/siRNA and chitosan/siRNA. Chitosan/siRNA samples containing 10% sucrose and Mirus/siRNA were added in different amounts into wells on 24 well plates to give the investigated concentrations of siRNA. The plates were then freeze dried and transfected for 48 hours with Raw cells. The cells were then incubated with LPS for 5 hours after which TNF-α was measured by ELISA (see figure 5). All samples in triplicates. Samples with a TNF-α production below the detection limit were treated as having concentrations at the detection limit of 40pg/ml standard deviation was not calculated for these samples (listed as N/A). TNF-α production was corrected for viability.**

| Sample | Averaged TNF-α Production pg/ml | Standard Deviation |
|---|---|---|
| Unstimulated Control | 96 | 2,4 |
| Stimulated Control | 1101 | 7,0 |
| Chitosan 50nm Match 10%S | 695 | 9,1 |
| Chitosan 50nm Mismatch 10%S | 1264 | 9,0 |
| Chitosan 25nm Match 10%S | 865 | 23,1 |
| Chitosan 25nm Mismatch 10%S | 1365 | 52,1 |
| Mirus 50nm Match | 200 | N/A |
| Mirus 50nm Mismatch | 264 | 8,4 |
| Mirus 25nm Match | 114 | N/A |
| Mirus 25nm Mismatch | 633 | 8,4 |

### Cytotoxicity Studies

We studied the viability of our two cell lines in the presence of the freeze-dried reagents. In the H1299 cell line no changes in viability was observed for chitosan/siRNA at low sucrose concentration (<=1 %) chitosan/siRNA, decreasing to about 90% at higher sucrose concentrations (=>5 %; Fig. 4A). In contrast, the Trans-IT TKO sample exhibited significantly lower viability of approximately 60 %.

The viability of Raw cells was significantly more sensitive to the transfection protocol than the H1299 cells (Fig. 4B). At 50 nM, all samples tested reduced the viability to below 40 % with the Trans-IT TKO sample reducing it to below 20 %. The viability increased slightly at lower concentrations (25 nM) of siRNA for both systems (from 33 % to 38 % with chitosan, from 18 % to 35 % with Trans-IT TKO). We conclude that the tested lipid formulation is significantly more toxic than chitosan particles.

### Discussion

One advantage of our chitosan-based siRNA delivery system compared to systems based on PEI is that PEI has been shown to be toxic in vitro and in vivo. Additionally, chitosan is a natural highly biodegradable polymer while PEI needs to be extensively chemically modified to achieve this. We found that in both cell lines the level of toxicity was higher with our lipid formulations than with our chitosan formulations. This is in line with the observation that cationic lipids induce toxicity by inhibiting important proteins like PKC.

Interestingly, our two formulations induced opposite unspecific effects of TNF-α production in macrophages. Our mismatched siRNA chitosan formulations increased TNF-α production slightly while our lipid formulation decreased TNF-α production. This finding underlines the importance of using multiple transfection reagents in knockdown studies because of the unpredictable off-target effects of transfection reagents. Our finding that chitosan stimulates TNF-α production supports previous findings that chitosan may stimulate the immune response to some extent. The use of specific siRNA, however, overcomes this.

Our chitosan system offers more rapid and convenient transfection, compared to current protocols where reagents are prepared, mixed and complexes must form (> 30 min). This is of huge benefit for applications requiring large amounts of transfection complexes such as in high throughput screening of knockdown of hundreds or thousands of genes.

The finding that surfaces coated with our freeze-dried formulations were capable of silencing TNF-α production in neighbouring macrophages could be utilized in implant technology and regenerative medicine. Macrophages are important mediators of the foreign body response, a response that triggers the immune system to react to non-self antigens and tissue damage. Since the TNF-α production is a prominent cytokine in this response, implants and tissue engineering scaffolds coated with our TNF-α directed formulation could be made more biocompatible by decreasing inflammatory responses. Work is currently being performed in our laboratory to evaluate the application of this strategy to tissue engineering.

### Conclusion

We have shown that chitosan can be used to formulate easy-to-use freeze-dried siRNA transfection reagents capable of efficient knockdown of different targets in two different cell lines. We have also shown that this system can be stored for extended periods. The system shows lower toxicity than lipid formulations. Finally the system offer the possibility to advance the fields of RNAi based high throughput screening, surface mediated siRNA delivery for implants as well as storage of lipid and chitosan-based siRNA therapeutics.

### Example 2

One ml 0.8mg/ml chitosan (Mw = 100kDa, DA = 80%) solution in 200mM sodium acetate buffer (pH 5,5) was added 20µl 100µM Cy3 labelled siRNA over 1 minute whilst stirring. The stirring was discontinued after 1 hour and 200µl 60% sucrose was added and the solution mixed with a pipette. Porous poly-ε-caprolactone scaffolds (size = 1mm³), designed for tissue engineering (where are these from?), were immersed completely in this solution for 10 min after which they were frozen at -20°C for 24 hours and freeze dried at -20°C and 100mT for 3 days.

Scaffolds without (Fig. 5A) and with (Fig. 5B) chitosan/siRNA particles were then visualized with scanning electron microscopy. Comparing scaffolds with and without chitosan/siRNA particles showed that the particles had been deposited on the scaffold walls as a coat covering surface features visible in the scaffold without particles.

We then added water to a coated scaffold and visualized the scaffold with fluorescence confocal microscopy (Fig. 6A). The siRNA (appearing brightly) could be seen adhering to the walls (appearing darker) indicating that the particles remained bound to the scaffold.

1000000 rat mesenchymal stem cells expressing enhanced fluorescent protein were added in 1 ml complete growth media to dry coated scaffolds and after 24 hours the cells were visualized using confocal microscopy (Fig. 6B). We observed that some of the siRNA had been taken up (appearing brightest) by the cells (appearing bright) adhering to the scaffolds walls (appearing darker) indicating successful transfection.

We conclude that by freeze drying chitosan/siRNA particles onto implants such as tissue engineering scaffolds we can get successful transfection of the cells growing on the implant. If a siRNA against an inflammatory cytokine (such as "tumor necrosis factor alpha" (TNF-α)1 was included in the chitosan particles coated onto a scaffold or any other type of implant it could probably reduce the inflammation associated with the implantation of such implants. Alternatively if a siRNA against a protein that represses a stem cell differentiation pathway (such as "BCL-2 like 2" (BCL2L2)2 or "growth-arrest specific gene 6" (GAS-6)3) was included in chitosan particles coated onto a scaffold or any other type of implant it could probably induce or enhance the differentiation of stem cells seeded onto these implants to a specific cell type.

### Example 3

One ml 800µg/ml chitosan (Mw = 100kDa, DA = 80%) solution in 200mM sodium acetate buffer (pH 5,5) was added 20µl 100µM fluorescently labelled anti RhoA siRNA over 1 minute while stirring. The stirring was discontinued after 1 hour and 200µl 60% sucrose was added and the solution mixed with a pipette. 7,6µl of the solution was placed in the middle of wells on 24 well plates. These plates were then frozen at -20°C for 24 hours and freeze dried at -20°C and 100mT for 3 days.

The plates were then thawed to room temperature for 10min during which the plate lid was taped onto the plate bottom using standard office tape. The plates were then frozen to -20°C for 2 days, shipped at room temperature for 1 day and frozen to -20°C for 3 days. The plates were then used by adding 250µl complete growth media containing 100000 cells (PC12 cells, Schwann cells and fibroblasts) to each well. After 1 day uptake of the siRNA was evaluated using fluorescence microscopy and phase contrast microscopy (Fig. 7). The siRNA was taken up in all three cell types indicating successful transfection.

We conclude that by freeze drying chitosan/siRNA particles in wells on micro well plates we can create an easy-to-use (1 step) transfection plate that gives a high ' rate of transfection in multiple cell types including neuronal cells. The chitosan/siRNA particles in the plates can survive being thawed and refrozen multiple times as well as being shipped to overseas customers.

## Claims

1. A dehydrated nanoparticle comprising a siRNA and chitosan.

2. The nanoparticle of claim 1 comprising less than 1 % water w/w.

3. The nanoparticle of any of the preceding claims being prepared by lyophilisation.

4. The nanoparticle of any of the preceding claims being included into a larger controlled release particle, an implantable drug depot or a biodegradable scaffold for tissue engineering.

5. The nanoparticle of any of the preceding claims being dried onto a surface.

6. The nanoparticle of any of the preceding claims being prepared by a method comprising
a. Providing a chitosan solution
b. Providing an siRNA solution
c. Mixing the solution of step a with the solution of step b
d. Incubating the solution of step c under conditions of complex formation such that chitosan/siRNA nanoparticles form.

7. The nanoparticle of any of the preceding claims, wherein the chitosan/siRNA nanoparticle does not comprise an initial crosslinker.

8. The nanoparticle of any of the preceding claims, wherein the siRNA solution comprises siRNA at a concentration lower than 100 µM.

9. The nanoparticle of any of the preceding claims, where the nanoparticle with loosely bound chitosan is for mucosal delivery or for systemic delivery.

10. The nanoparticle of any of the preceding claims, wherein the concentration of the siRNA is higher than 100 µM.

11. The nanoparticle of any of the preceding claims, wherein the chitosan concentration is less than 250 µg/ml.

12. The nanoparticle of any of the preceding claims, wherein the size of the particle is between 10 and 500 nm.

13. A surface comprising a nanoparticle according to any of claims 1-12 wherein the nanoparticle has been dried onto the surface.

14. A powder of the nanoparticles of any of claims 1-12.

15. A pharmaceutical composition comprising the nanoparticle of any of claims 1-12.

## Patentansprüche

1. Dehydriertes Nanopartikel, umfassend eine siRNA und Chitosan.

2. Nanopartikel nach Anspruch 1, umfassend weniger als 1 Gew.-% Wasser.

3. Nanopartikel nach einem der vorhergehenden Ansprüche, das durch Lyophilisierung hergestellt wird.

4. Nanopartikel nach einem der vorhergehenden Ansprüche als Teil eines größeren Teilchens mit kontrollierter Freigabe, eines implantierbaren Arzneimitteldepots oder eines biologisch abbaubaren Gerüsts für Gewebekonstruktionen (Tissue Engineering).

5. Nanopartikel nach einem der vorhergehenden Ansprüche, das auf eine Fläche aufgetrocknet wird.

6. Nanopartikel nach einem der vorhergehenden Ansprüche, das durch ein Verfahren hergestellt wird, umfassend
a. Bereitstellen einer Chitosanlösung
b. Bereitstellen einer siRNA-Lösung
c. Mischen der Lösung aus Schritt a mit der Lösung aus Schritt b
d. Inkubieren der Lösung aus Schritt c unter Bedingungen für komplexe Ausbildungen, sodass sich Chitosan-/siRNA-Nanopartikel bilden.

7. Nanopartikel nach einem der vorhergehenden Ansprüche, wobei das Chitosan-/siRNA-Nanopartikel keinen anfänglichen Vernetzer umfasst.

8. Nanopartikel nach einem der vorhergehenden Ansprüche, wobei die siRNA-Lösung siRNA in einer Konzentration von weniger als 100 µM umfasst.

9. Nanopartikel nach einem der vorhergehenden Ansprüche, wobei das Nanopartikel mit locker gebundenem Chitosan für die Abgabe an die Schleimhaut oder die systemische Abgabe vorgesehen ist.

10. Nanopartikel nach einem der vorhergehenden Ansprüche, wobei die Konzentration von siRNA mehr als 100 µM beträgt.

11. Nanopartikel nach einem der vorhergehenden Ansprüche, wobei die Konzentration von Chitosan mehr als 250 µg/ml beträgt.

12. Nanopartikel! nach einem der vorhergehenden Ansprüche, wobei die Größe des Partikels zwischen 10 und 500 nm liegt.

13. Eine Fläche, umfassend ein Nanopartikel nach einem der Ansprüche 1-12, wobei das Nanopartikel auf die Fläche aufgetrocknet ist.

14. Pulver der Nanopartikel nach einem der Ansprüche 1-12.

15. Pharmazeutische Zusammensetzung, umfassend das Nanopartikel nach einem der Ansprüche 1-12.

## Revendications

1. Nanoparticule déshydratée comprenant un siRNA et du chitosane.

2. La nanoparticule de la revendication 1 comprenant moins de 1 % d'eau p/p.

3. La nanoparticule de l'une quelconque des revendications précédentes étant préparée par lyophilisation.

4. La nanoparticule de l'une quelconque des revendications précédentes étant incluse dans une particule à libération contrôlée de plus grande taille, un dépôt de médicament implantable ou un support biodégradable pour ingénierie tissulaire.

5. La nanoparticule de l'une quelconque des revendications précédentes étant séchée sur une surface.

6. La nanoparticule de l'une quelconque des revendications précédentes étant préparée par une méthode comprenant les étapes suivantes
a. apport d'une solution de chitosane,
b. apport d'une solution de siRNA,
c. mélange de la solution de l'étape a avec la solution de l'étape b,
d. incubation de la solution de l'étape c dans des conditions de complexation telles que des nanoparticules de chitosane/siRNA se forment.

7. La nanoparticule de l'une quelconque des revendications précédentes, dans laquelle la nanoparticule de chitosane/siRNA ne comprend pas de réticulant initial.

8. La nanoparticule de l'une quelconque des revendications précédentes, dans laquelle la solution de siRNA comprend du siRNA à une concentration inférieure à 100 µM.

9. La nanoparticule de l'une quelconque des revendications précédentes, dans laquelle la nanoparticule avec du chitosane faiblement lié est destinée à une administration par voie muqueuse ou à une administration par voie générale.

10. La nanoparticule de l'une quelconque des revendications précédentes, dans laquelle la concentration du siRNA est supérieure à 100 µM.

11. La nanoparticule de l'une quelconque des revendications précédentes, dans laquelle la concentration de chitosane est inférieure à 250 µg/ml.

12. La nanoparticule de l'une quelconque des revendications précédentes, dans laquelle la taille de la particule est comprise entre 10 et 500 nm.

13. Surface comprenant une nanoparticule selon l'une quelconque des revendications 1 à 12 dans laquelle la nanoparticule a été séchée sur la surface.

14. Poudre de la nanoparticule de l'une quelconque des revendications 1 à 12.

15. Composition pharmaceutique comprenant la nanoparticule de l'une quelconque des revendications 1 à 12.
